(19) Europäisches Patentamt
European Patent Office
Office européen des brevets

(11) Veröffentlichungsnummer: **0 061 053**
**B1**

(12) **EUROPÄISCHE PATENTSCHRIFT**

(45) Veröffentlichungstag der Patentschrift: **22.08.84**

(51) Int. Cl.³: **C 07 C 69/747**, C 07 C 67/08, A 01 N 53/00

(21) Anmeldenummer: **82101803.3**

(22) Anmeldetag: **08.03.82**

(54) **3-Alken-1-yl-2,2-dimethyl-cyclopropancarbonsäure-(4-fluor-3-phenoxy-benzyl)-ester, Verfahren zu ihrer Herstellung und ihre Verwendung in Schädlingsbekämpfungsmitteln.**

(30) Priorität: **19.03.81 DE 3110725**

(43) Veröffentlichungstag der Anmeldung: **29.09.82 Patentblatt 82/39**

(45) Bekanntmachung des Hinweises auf die Patenterteilung: **22.08.84 Patentblatt 84/34**

(84) Benannte Vertragsstaaten: **BE CH DE FR GB IT LI NL**

(56) Entgegenhaltungen:
**DE - A - 2 709 264**
**US - A - 3 666 789**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 WUppertal 1 (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**
Erfinder: **Behrenz, Wolfgang, Dr., Untergruendemich 14, D-5063 Overath (DE)**
Erfinder: **Stendel, Wilhelm, Dr., In den Birken 55, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 061 053 B1

ACTORUM AG

**Beschreibung**

Die Erfindung betrifft neue 3-Alken-1-yl-2,2-dimethylcyclopropancarbonsäure-(4-fluor-3-phenoxybenzyl(ester, ein Verfahren zu ihrer Herstellung und ihre Verwendung in Schädlingsbekämpfungsmitteln, insbesondere in Insektiziden und Akariziden.

Es ist bekannt, dass bestimmte substituierte Cyclopropancarbonsäurephenoxybenzylester, wie z.B. 3-(2-Methylpropen-1-yl)-2,2-dimethylcyclopropancarbonsäure-(3-phenoxybenzyl)-ester (Phenothrin) und 3-(2-Methylpropen-1-yl)-2,2-dimethylcyclopropancarbonsäure-[3-(4-fluorphenoxy)-α-cyanobenzyl]ester insektizide und akarizide Eigenschaften aufweisen (vgl. GB-PS Nrn. 1243858 und 1549462). Ausserdem ist bekannt, dass 3-(2-Methylpropen-1-yl)-2,2-dimethylcyclopropancarbonsäure-(3-phenoxy-4-fluor)-α-cyanobenzylester insektizide Wirkung aufweist (vgl. DE-A Nr. 2709264).

Die Wirkung dieser Verbindungen ist jedoch insbesondere bei niedrigen Wirkstoffkonzentrationen und Aufwandmengen nicht immer zufriedenstellend. Es wurden nun neue 3-Alken-1-yl-2,2-dimethylcyclopropancarbonsäure-(4-fluor-3-phenoxybenzyl)ester der Formel I:

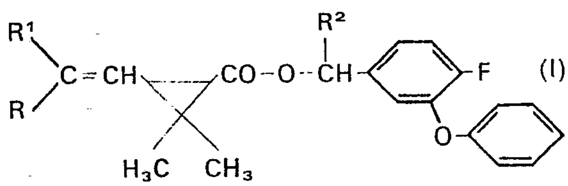

(I)

gefunden, in welcher

R für Alkyl oder Alkenyl steht.

$R^1$ für Wasserstoff oder Alkyl steht mit der Massgabe, dass R und $R^1$ verschieden sind, und

$R^2$ für Wasserstoff, Cyano, Alkyl, Alkenyl oder Alkinyl, letztere jeweils mit bis zu 4 Kohlenstoffatomen, steht.

Die allgemeine Formel I schliesst die verschiedenen möglichen Stereoisomeren und optischen Isomeren sowie deren Mischungen mit ein.

Man erhält die neuen Verbindungen der Formel I, wenn man 3-Alken-1-yl-2,2-dimethylcyclopropancarbonsäuren der Formel II:

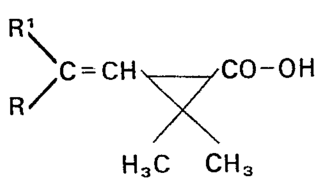

(II)

in welcher

R und $R^1$ die oben angegebene Bedeutung haben,

oder reaktionsfähige Derivate derselben, mit 4-Fluor-3-phenoxybenzylalkoholen der Formel III:

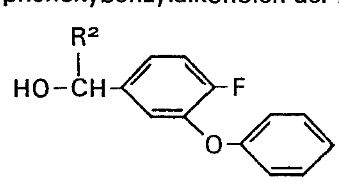

(III)

in welcher

$R^2$ die oben angegebene Bedeutung hat,

oder mit reaktionsfähigen Derivaten derselben, gegebenenfalls in Gegenwart von Säureakzeptoren und/oder Katalysatoren und gegebenenfalls unter Verwendung von Verdünnungsmitteln umsetzt.

Die neuen 3-Alken-1-yl-2,2-dimethylcyclopropancarbonsäure-(4-fluor-3-phenoxybenzyl)-ester der Formel I zeichnen sich durch hohe pestizide Wirksamkeit aus.

Überraschenderweise zeigen die erfindungsgemässen Verbindungen der Formel I eine erheblich höhere insektizide und akarizide Wirkung als aus dem Stand der Technik bekannte Verbindungen analoger Konstitution und gleicher Wirkungsrichtung.

Die Erfindung betrifft vorzugsweise Verbindungen der Formel I, in welcher

R für $C_1$ bis $C_{25}$-Alkyl oder $C_2$ bis $C_{16}$-Alkenyl steht,

$R^1$ für Wasserstoff oder Methyl steht mit der Massgabe, dass R und $R^1$ verschieden sind, und

$R^2$ für Waserstoff oder Cyano steht.

Besonders bevorzugt sind Verbindungen der Formel I, in welcher

R für $C_1$ bis $C_{15}$-Alkyl oder $C_2$ bis $C_4$-Alkenyl steht,

$R^1$ für Wasserstoff oder Methyl steht, wobei R und $R^2$ verschieden sind, und

$R^2$ für Wasserstoff oder Cyano steht.

In einer bevorzugten Variante a des Herstellungsverfahrens für die Verbindungen der Formel I werden 3-Alken-1-yl-2,2-dimethylcyclopropancarbonsäurechloride der Formel IIa:

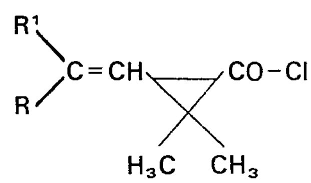

(IIa)

in welcher

R und $R^1$ die oben angegebene Bedeutung haben,

mit 4-Fluor-3-phenoxybenzylalkoholen der Formel III (oben) in Gegenwart von Säureakzeptoren und unter Verwendung von Verdünnungsmitteln umgesetzt.

In einer weiteren bevorzugten Verfahrensvariante b zur Herstellung von Verbindungen der Formel I, in welcher $R^2$ für Cyano steht, werden Säurechloride der Formel IIa (oben) mit 4-Fluor-3-phenoxybenzaldehyd der Formel IV:

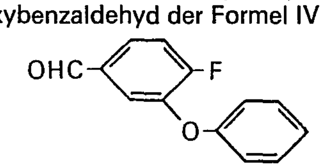

(IV)

und wenigstens der äquimolaren Menge eines Alkalicyanids (Natrium- oder Kaliumcyanid) in Gegenwart von Wasser und eines mit Wasser praktisch nicht mischbaren Verdünnungsmittels und gegebenenfalls in Gegenwart eines Katalysators umgesetzt.

Als weitere reaktionsfähige Derivate der Carbonsäure der Formel II sind deren Niederalkylester zu nennen, welche mit Alkoholen der Formel III nach üblichen Methoden umgesetzt werden können.

Alkali-, Erdalkali- oder Ammoniumsalze der Carbonsäure II können mit Benzylhalogeniden, welche sich von den Benzylalkoholen der Formel III ableiten, ebenfalls zu Verbindungen der Formel I umgesetzt werden.

Verwendet man als Ausgangsstoffe beispielsweise 3-Propen-1-yl-2,2-dimethylcyclopropancarbonsäurechlorid und bei der Verfahrensvariante a 4-Fluor-3-phenoxybenzylalkohol bzw. bei Variante b Natriumcyanid und 4-Fluor-3-phenoxybenzaldehyd, so können die bei den beiden Verfahrensvarianten ablaufenden Reaktionen durch folgende Formelschemata skizziert werden:

(a)

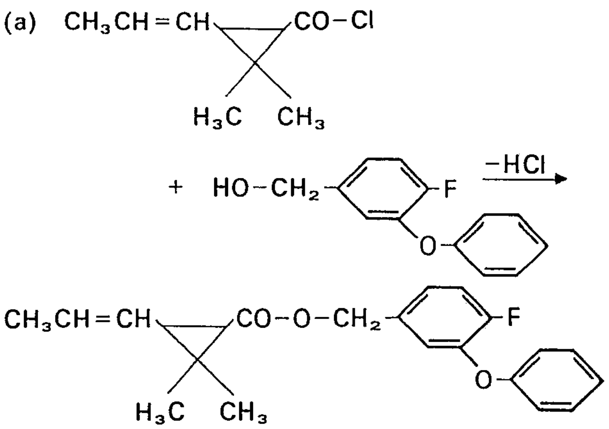


(b)

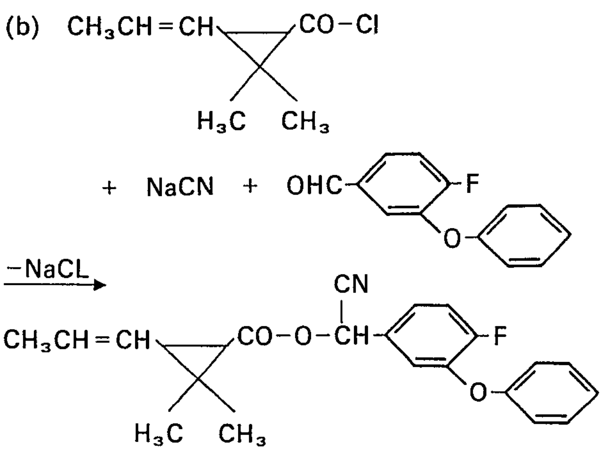


Die als Ausgangsstoffe zu verwendenden 3-Alken-1-yl-2,2-dimethylcyclopropancarbonsäuren sind durch Formel II, die entsprechenden Säurechloride durch Formel IIa definiert. Vorzugsweise stehen in diesen Formeln R und $R^1$ für diejenigen Reste, welche bei der Definition der entsprechenden Reste R und $R^1$ in Formel I als bevorzugt genannt wurden.

Als Beispiele für die Ausgangsverbindungen der Formeln II und IIa seien genannt:

3-Propen-1-yl-, 3-Buten-1-yl-, 3-Penten-1-yl-, 3-Hexen-1-yl-, 3-Hepten-1yl-, 3-Octen-1-yl-, 3-Nonen-1-yl, 3-Decen-1-yl-, 3-Undecen-1-yl-, 3-Dodecen-1-yl-, 3-Tridecen-1-yl-, 3-Tetradecen-1-yl-, 3-Pentadecen-1-yl-, 3-Hexadecen-1-yl-, 3-Heptadecen-1-yl-, 3-Octadecen-1-yl-, 3-(2-Methyl)buten-1-yl-, 3-(2-Methyl)penten-1-yl-, 3-(2-Methyl)hexen-1-yl-, 3-(2-Methyl)-hepten-1-yl-, 3-(2-Methyl)octen-1-yl-, 3-(2-Methyl)nonen-1-yl-, 3-(2-Methyl)decen-1-yl-, 3-(2-Methyl)undecen-1-yl-, 3-(2-Methyl)dodecen-1-yl-, 3-(2-Methyl)tridecen-1-yl-, 3-(2-Methyl)tetradecen-1-yl-, 3-(2-Methyl)pentadecen-1-yl-, 3-(2-Methyl)hexadecen-1-yl-, 3-(2-Methyl)heptadecen-1-yl-, 3-(1-Methyl)octadecen-1-yl-, 3-Butadien-1,3-yl-, 3-Pentadien-1,3-yl-, 3-(4-Methyl)pentadien-1,3-yl- und 3-(4-Methyl)penten-1-yl-2,2-dimethylcyclopropancarbonsäure sowie die entsprechenden Säurechloride.

3-Alken-1-yl-2,2-dimethylcyclopropancarbonsäuren der Formel II, entsprechende Säurechloride der Formel IIa oder entsprechende Niederalkylester sind bekannt und/oder können nach an sich bekannten Verfahren hergestellt werden (vgl. GB-PS Nr. 1413491 und US-PS Nrn. 3847944, 3954814, 3998868).

Die weiter als Ausgangsstoffe zu verwendenden 4-Fluor-3-phenoxybenzylalkohole sind durch Formel III definiert. Vorzugsweise steht darin $R^2$ für Wasserstoff oder Cyano.

Als Beispiele seien 4-Fluor-3-phenoxybenzylalkohol und 4-Fluor-3-phenoxy-α-cyanobenzylalkohol genannt.

Verbindungen der Formel III sind bereits bekannt (vgl. US-PS Nr. 4218469).

Der als Ausgangsstoff verwendbare 4-Fluor-3-phenoxybenzaldehyd der Formel IV ist ebenfalls bekannt (vgl. US-PS Nr. 4218469).

Das Verfahren zur Herstellung der neuen Verbindungen der Formel I wird in allen Varianten vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Die Variante a des erfindungsgemässen Verfahrens wird vorzugsweise in Gegenwart von Säureakzeptoren durchgeführt. Als Säureakzeptoren können die üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Diazabicyclooctan, Diazabicyclononen und Diazabicycloundecen.

Die Variante b des erfindungsgemässen Verfahrens wird in Gegenwart von Wasser und eines der oben genannten organischen Lösungsmittel, soweit mit Wasser nicht mischbar, durchgeführt. Hierfür sind insbesondere die oben genannten Kohlenwasserstoffe geeignet.

Als Katalysatoren werden bei der Verfahrensvariante b vorzugsweise Verbindungen verwendet, welche zum Transfer von Anionen aus Wasser in organische Lösungsmittel geeignet sind. Beispiele hierfür sind Benzyltriethylammoniumhydrogensulfat, Triethylbenzylammoniumchlorid, Tetrabutylammoniumbromid und Methyltrioctylammoniumchlorid (Aliquat 336).

In allen Verfahrensvarianten kann die Reaktionstemperatur innerhalb eines grösseren Bereichs variiert werden. Im allgemeinen arbeitet man zwischen 0 und 100° C, vorzugsweise bei 10 bis 50° C.

Das erfindungsgemässe Verfahren wird im allgemeinen bei Normaldruck durchgeführt. Zur Durchführung des erfindungsgemässen Verfahrens werden die Ausgangsstoffe gewöhnlich in äquimolaren Mengen eingesetzt. Ein Überschuss der einen oder anderen Reaktionskomponente bringt keine wesentlichen Vorteile. Die Ausgangsstoffe werden in geeigneten Verdünnungsmitteln zusammen gegeben und, gegebenenfalls nach Zugabe eines Säureakzeptors und/oder eines Katalysators, bis zum Reaktionsende gerührt.

Die Aufarbeitung kann nach üblichen Methoden durchgeführt werden, beispielsweise indem man das Reaktionsgemisch gegebenenfalls mit Wasser und/oder einem mit Wasser nicht mischbaren organischen Lösungsmittel, wie z.B. Toluol, verdünnt, die organische Phase abtrennt, mit Wasser wäscht, trocknet, filtriert und vom Filtrat das Lösungsmittel unter vermindertem Druck und bei mässig erhöhter Temperatur sorgfältig abdestilliert (andestilliert).

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der *Isopoda* z.B. *Oniscus asellus, Armadillidium vulgare, Porcellio scaber.*

Aus der Ordnung der *Diplopoda* z.B. *Blaniulus guttulatus.*

Aus der Ordnung der *Chilopoda* z.B. *Geophilus carpophagus, Scutigera spec.*

Aus der Ordnung der *Symphyla* z.B. *Scutigerella immaculata.*

Aus der Ordnung der *Thysanura* z.B. *Lepisma saccharina.*

Aus der Ordnung der *Collembola* z.B. *Onychiurus armatus.*

Aus der Ordnung der *Orthoptera* z.B. *Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.*

Aus der Ordnung der *Dermaptera* z.B. *Forficula auricularia.*

Aus der Ordnung der *Isoptera* z.B. *Reticulitermes spp.*

Aus der Ordnung der *Anoplura* z.B. *Phylloxera vastatrix, Pemphigus spp., Pediculus humanus corporis, Haematopinus spp., Linognathus spp.*

Aus der Ordnung der *Mallophaga* z.B. *Trichodectes spp., Damalinea spp.*

Aus der Ordnung der *Thysanoptera* z.B. *Hercinothrips femoralis, Thrips tabaci.*

Aus der Ordnung der *Heteroptera* z.B. *Eurygaster spp., Dysdercus intermedius, Piesma quadrata, Cimex lectularius, Rhodnius prolixus, Triatoma spp.*

Aus der Ordnung der *Homoptera* z.B. *Aleurodes brassicae, Bemisia tabaci, Trialerodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.*

Aus der Ordnung der *Lepidoptera* z.B. *Pectinophora gossypiela, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubillis, Ephestia kuehniella, Galleria mellonella, Cacoecia podana, Capua reticulana, Choristoneura fumiferana, Clysia ambiguella, Homona magnanima, Tortrix viridana.*

Aus der Ordnung der *Coleoptera* z.B. *Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.*

Aus der Ordnung der *Hymenoptera* z.B. *Diprion*

*spp., Hoplocampa spp., Lasius spp., Monomorium pharaonis, Vespa spp.*

Aus der Ordnung der *Diptera* z.B. *Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxs spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.*

Aus der Ordnung der *Siphonaptera* z.B. *Xenopsylla cheopis, Ceratophyllus spp.*

Aus der Ordnung der *Arachnida* z.B. *Scorpio maurus, Latrodectus mactans.*

Aud der Ordnung der *Acarina* z.B. *Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.*

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine, wie Calcit, Marmor, Bims, Sepiolith, Dolomit sowie synthetische Granulat aus anorganischen und organischen Mehlen sowie Granulate aus organischem Material, wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen/Fettsäure/Ester, Polyoxyethylen/Fettalkohol/Ether, z.B. Alkylarylpolyglykolether, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat.

Es können Farbstoffe wie anorganische Pigmente, z.B. Eisenoxid, Titanoxid, Ferrocyanblau und organische Farbstoffe, Stoffe, wie Alizarin-, Azo- und Metallphthalocyaninfarbstoffe, Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-%, liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und

Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem veterinärmedizinischen Gebiet.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens *(pour-on and spot-on)* und des Einpuders sowie durch parenterale Anwendung in Form beispielsweise der Injektion.

*Herstellungsbeispiele*

*Beispiel 1:*

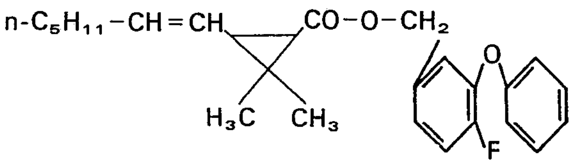

Zu einer Lösung von 8,8 g (0,04 mol) 4-Fluor-3-phenoxybenzylalkohol und 4,2 g (0,041 mol) Triethylamin in 80 ml Toluol tropft man eine Lösung von 2-[1-Hepten(1)yl]-3,3-dimethylcyclopropancarbonsäurechlorid in 10 ml Toluol. Das Reaktionsgemisch wird 18 h bei Raumtemperatur nachgerührt und dann zweimal mit je 50 ml Wasser gewaschen. Die organische Phase wird über Natriumsulfat getrocknet und im Vakuum eingedampft; den Rückstand destilliert man im Hochvakuum an. Man erhält so 15,2 g (93% der Theorie) 2-[1-Hepten(1)yl]-3,3-dimethylcyclopropancarbonsäure-(4-fluor-3-phenoxybenzyl)ester in Form eines gelben Öles mit dem Brechungsindex $n_D^{20}$: 1,5302.

*Beispiel 2:*

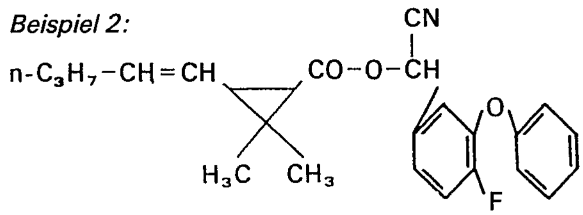

Eine Mischung aus 40 ml Toluol, 6 g (0,028 mol) 4-Fluor-3-phenoxybenzaldehyd und 0,3 g Triethylbenzylammoniumchlorid wird erst mit einer Lösung von 2,05 g (0,042 mol) Natriumcyanid in 5 ml Wasser und dann mit einer Lösung von 5,6 g (0,028 mol) 2-[1-Penten(1)yl]-3,3-dimethylcyclopropancarbonsäurechlorid in 6 ml Toluol versetzt. Anschliessend rührt man das Reaktionsgemisch 18 h bei Raumtemperatur nach und wäscht dann zweimal mit je 50 ml Wasser. Die organische Phase wird über Natriumsulfat getrocknet und im Vakuum eingedampft; den Rückstand destilliert man im Hochvakuum an. Zurück bleiben 10 g (88% der Theorie) 2-[1-Penten(1)-yl]-3,3-dimethylcyclopropancarbonsäurecyano-(4-fluor-3-phenoxybenzyl)ester in Form eines gelben, zähen Öles mit dem Brechungsindex $n_D^{20}$: 1,5383.

Analog einem der Beispiele 1 oder 2 können die folgenden Verbindungen der Formel:

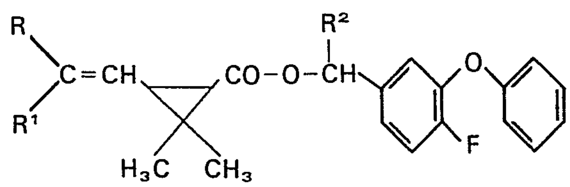

hergestellt werden:

| Beispiel Nr. | R | R¹ | R² | Ausbeute (% der Theorie) | Brechungs-index |
|---|---|---|---|---|---|
| 3 | $n\text{-}C_5H_{11}$ | H | CN | 84 | $n_D^{20}$:1,5312 |
| 4 | $C_2H_5$ | H | CN | 92 | $n_D^{20}$:1,5395 |
| 5 | $CH_3$ | H | CN | 91 | $n_D^{19}$:1,5448 |
| 6 | $n\text{-}C_4H_9$ | H | CN | 73 | $n_D^{19}$:1,5357 |
| 7 | $n\text{-}C_6H_{13}$ | H | CN | 73 | $n_D^{19}$:1,5293 |
| 8 | $n\text{-}C_{15}H_{31}$ | H | CN | 97 | $n_D^{22}$:1,5148 |
| 9 | $(CH_3)_2C{=}CH$ | H | CN | 70 | $n_D^{25}$:1,5583 |
| 10 | $n\text{-}C_7H_{15}$ | H | CN | 95 | $n_D^{25}$:1,5239 |
| 11 | $n\text{-}C_8H_{17}$ | H | CN | 91 | $n_D^{23}$:1,5227 |
| 12 | $n\text{-}C_5H_{11}$ | $CH_3$ | CN | 89 | $n_D^{19}$:1,5298 |
| 13 | $(CH_3)_2CH{-}CH_2$ | H | CN | | |
| 14 | $CH_3$ | H | H | | |
| 15 | $C_2H_5$ | H | H | | |
| 16 | $n\text{-}C_3H_7$ | H | H | | |
| 17 | $n\text{-}C_4H_9$ | H | H | | |
| 18 | $n\text{-}C_4H_9$ | $CH_3$ | CN | | |
| 19 | $n\text{-}C_3H_7$ | $CH_3$ | CN | 99 | $n_D^{21}$:1,5355 |
| 20 | $C_2H_5$ | $CH_3$ | CN | | |
| 21 | $(CH_3)_2CH{-}CH_2{-}CH_2$ | H | CN | | |
| 22 | $n\text{-}C_6H_{13}$ | $CH_3$ | CN | | |
| 23 | $n\text{-}C_5H_{11}$ | $CH_3$ | H | | |

Die als Ausgangsmaterialien zu verwendenden substituierten Cyclopropancarbonsäurechloride können z.B. wie folgt hergestellt werden:

*Beispiel*

*1. Stufe:*

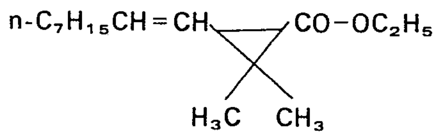

Zu einer Suspension von 68,3 g (0,15 mol) n-Octyltriphenylphosphoniumbromid in 300 ml absolutem Tetrahydrofuran tropft man unter Luftausschluss bei 0-5°C 64,5 g einer 15%igen Lösung von Butyllithium in n-Hexan. Nach 1 h gibt man langsam 25,5 g (0,15 mol) 2-Formyl-3,3-dimethylcyclopropancarbonsäureethylester zu und rührt das Gemisch 18 h bei Raumtemperatur nach. Nach Zugabe von 400 ml Toluol und 300 ml Wasser wird geschüttelt, das Wasser abgetrennt und die organische Phase noch einmal mit 200 ml Wasser gewaschen. Dann trocknet man die Toluollösung über Natriumsulfat und destilliert das Lösungsmittel im Vakuum ab. Den Rückstand verrührt man mit 300 ml Petrolether (40-60°C), saugt vom ungelösten Triphenylphosphinoxid ab und dampft das Filtrat im Vakuum erneut ein. Der Rückstand wird im Vakuum destilliert. Man erhält so 22,6 g (57% der Theorie) 2-[1-Nonen(1)yl]-3,3-dimethylcyclopropancarbonsäureethylester mit dem Siedepunkt 90°C/1,33 Pa.

In analoger Weise können die folgenden Verbindungen der Formel:

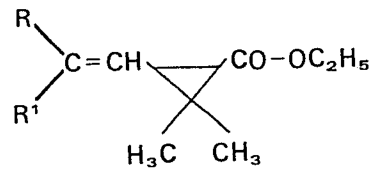

hergestellt werden:

| R | $R^1$ | Ausbeute (% der Theorie) | Siedpunkt (°C/Pa) |
|---|---|---|---|
| $n-C_5H_{11}$ | H | 60 | 92-95/66,5 |
| $n-C_3H_7$ | H | 56 | 72-75/40 |
| $C_2H_5$ | H | 59 | 108-110/1333 |
| $CH_3$ | H | 60 | 93-96/1333 |
| $n-C_4H_9$ | H | 48 | 63-65/1,33 |
| $n-C_6H_{13}$ | H | 54 | 92-94/2,66 |
| $n-C_{15}H_{31}$ | H | 57 | 170-175/13,3 |
| $(CH_3)_2C=CH$ | H | 66 | 70/13,3 |
| $n-C_8H_{17}$ | H | 65 | 110/13,3 |
| $n-C_5H_{11}$ | $CH_3$ | 57 | 90-93/26,6 |
| $(CH_3)_2CH-CH_2$ | H | | |
| $n-C_4H_9$ | $CH_3$ | | |
| $n-C_3H_7$ | $CH_3$ | 49 | 76/13,3 |
| $C_2H_5$ | $CH_3$ | | |
| $(CH_3)_2CH-CH_2-CH_2$ | H | | |
| $n-C_6H_{13}$ | $CH_3$ | | |

*2. Stufe:*

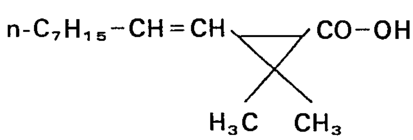

Eine Mischung aus 5,6 g (0,1 mol) Kaliumhydroxid, 45 ml Wasser, 45 ml Ethanol und 22 g (0,083 mol) 2-[1-Nonen(1)yl]-3,3-dimethylcyclopropancarbonsäureethylester wird 5 h unter Rückfluss gekocht, dann mit 150 ml Wasser versetzt und 2mal je 100 ml Ether extrahiert. Die wässerige Phase wird angesäuert und das Produkt mit Methylenchlorid (2mal je 100 ml) ausgeschüttelt. Nach Trocknen über Natriumsulfat destilliert man das Lösungsmittel im Vakuum ab. Zurück bleiben 17 g (86% der Theorie) 2-[1-Nonen(1)yl]-3,3-dimethylcyclopropancarbonsäure in Form eines gelben Öles.

In analoger Weise können die folgenden Verbindungen der Formel:

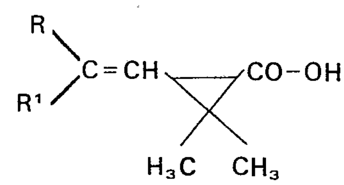

hergestellt werden.

| R | $R^1$ | Ausbeute (% der Theorie) |
|---|---|---|
| $n-C_5H_{11}$ | H | 93 |
| $n-C_3H_7$ | H | 92 |
| $C_2H_5$ | H | 77 |
| $CH_3$ | H | 86 |
| $n-C_4H_9$ | H | 80 |
| $n-C_6H_{13}$ | H | 82 |
| $n-C_{15}H_{31}$ | H | 85 |
| $(CH_3)_2C=CH$ | H | 99 |
| $n-C_8H_{17}$ | H | 84 |
| $n-C_5H_{11}$ | $CH_3$ | 86 |

| R | $R^1$ | Ausbeute (% der Theorie) |
|---|---|---|
| $(CH_3)_2CH-CH_2$ | H | |
| $n-C_4H_9$ | $CH_3$ | |
| $n-C_3H_7$ | $CH_3$ | |
| $C_2H_5$ | $CH_3$ | |
| $(CH_3)_2CH-CH_2-CH_2$ | H | |
| $n-C_6H_{13}$ | $CH_3$ | |

*3. Stufe:*

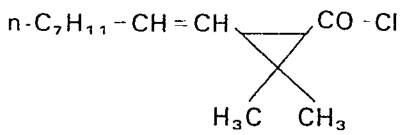

Zu einer Lösung von 16,8 g (0,07 mol) 2-[1-Nonen(1)yl]-3,3-dimethylcyclopropansäure in 80 ml Methylenchlorid gibt man 2 Tropfen Dimethylformamid und dann 11,2 g (0,095 mol) Thionylchlorid. Anschliessend kocht man die Mischung 1 h unter Rückfluss, destilliert das Lösungsmittel im Vakuum ab und destilliert den Rückstand im Vakuum. Man erhält auf diese Weise 16 g (89% der Theorie) 2-[1-Nonen(1)yl]-3,3-dimethylcyclopropancarbonsäurechlorid mit dem Siedepunkt 110° C/66,5 Pa.

In analoger Weise können die folgenden Verbindungen der Formel:

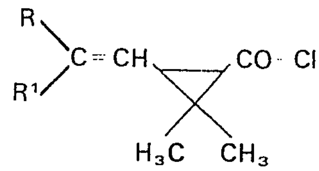

hergestellt werden:

| R | $R^1$ | Ausbeute (% der Theorie) | Siedepunkt (° C/Pa) |
|---|---|---|---|
| $n-C_5H_{11}$ | H | 83 | 70/13,3 |
| $n-C_3H_7$ | H | 85 | 48/6,65 |
| $C_2H_5$ | H | 84 | 55/66,5 |
| $CH_3$ | H | 86 | 51/133 |
| $n-C_4H_9$ | H | 91 | 65/40 |
| $n-C_6H_{13}$ | H | 82 | 85/26 |
| $n-C_{15}H_{31}$ | H | 91 | 191/53 |
| $(CH_3)_2C=CH$ | H | 85 | 81/26 |
| $n-C_8H_{17}$ | H | 83 | 105/1,33 |
| $n-C_5H_{11}$ | $CH_3$ | 84 | 84/13,3 |
| $(CH_3)_2CH-CH_2$ | H | | |
| $n-C_4H_9$ | $CH_3$ | | |
| $n-C_3H_7$ | $CH_3$ | 85 | 72/26 |
| $C_2H_5$ | $CH_3$ | | |
| $(CH_3)_2CH-CH_2-CH_2$ | H | | |
| $n-C_6H_{13}$ | $CH_3$ | | |

*Beispiel A:*

Drosophila-*Test*

Lösungsmittel: 3 Gew.-Teile Aceton

Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschte Konzentration.

1 $cm^3$ der Wirkstoffzubereitung wird auf eine Filterpapierscheibe (7 cm Durchmesser) aufpipettiert. Man legt diese nass auf die Öffnung eines Glasgefässes, in dem sich 50 Taufliegen *(Drosophila melanogaster)* befinden und bedeckt es mit einer Glasplatte.

Nach der gewünschten Zeit bestimmt man die Abtötung in Prozent. Dabei bedeutet 100%, dass alle Fliegen abgetötet wurden; 0% bedeutet, dass keine Fliegen abgetötet wurden.

Bei diesem Test zeigen z.B. die folgenden Verbindungen der Herstellungsbeispiele überlegene Wirksamkeit gegenüber dem Stand der Technik: 5, 4, 2, 6, 3, 12, 7, 10, 11.

*Beispiel B:*

Phaedon-*Larven-Test*

Lösungsmittel: 3 Gew.-Teile Aceton

Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschte Konzentration.

Kohlblätter *(Brassica oleracea)* werden durch Tauchen in die Wirkstoffzubereitung der gewünschten Konzentration behandelt und mit Meerrettichblattkäfer-Larven *(Phaedon cochleariae)* besetzt, solange die Blätter noch feucht sind.

Nach der gewünschten Zeit wird die Abtötung in Prozent bestimmt. Dabei bedeutet 100%, das alle Käfer-Larven abgetötet wurden; 0% bedeutet, dass keine Käfer-Larven abgetötet wurden.

Bei diesem Test zeigen z.B. die folgenden Verbindungen der Herstellungsbeispiele überlegene Wirksamkeit gegenüber dem Stand der Technik: 5, 9, 4, 2, 6, 12, 7, 10, 11, 8.

*Beispiel C:*

Tetranychus-*Test (resistent)*
Lösungsmittel: 3 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel und der angegebenen Menge Emulgator und verdünnt das Konzentrat mit Wasser auf die gewünschte Konzentration.

Bohnenpflanzen *(Phaseolus vulgaris)*, die stark von allen Entwicklungsstadien der gemeinen Spinnmilbe oder Bohnenspinnmilbe *(Tetranychus urticae)* befallen sind, werden durch Tauchen in die Wirkstoffzubereitung der gewünschten Konzentration behandelt.

Nach der gewünschten Zeit wird die Abtötung in Prozent bestimmt. Dabei bedeutet 100%, dass alle Spinnmilben abgetötet wurden; 0% bedeutet, dass keine Spinnmilben abgetötet wurden.

Bei diesem Test zeigen z.B. die folgenden Verbindungen der Herstellungsbeispiele überlegene Wirksamkeit gegenüber dem Stand der Technik: 4, 2, 6, 12.

*Beispiel D:*

*Grenzkonzentrations-Test/Bodeninsekten*
Testinsekt: *Phorbia antigua*-Maden (im Boden)
Lösungsmittel: 3 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in Teilen pro Million (ppm = mg/l) angegeben wird. Man füllt den Boden in Töpfe und lässt diese bei Raumtemperatur stehen.

Nach 24 h werden die Testtiere in den behandelten Boden gegeben und nach weiteren 2 bis 7 d wird der Wirkungsgrad des Wirkstoffs durch Auszählen der toten und lebenden Testinsekten in Prozent bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 3, 2, 4, 5.

*Beispiel E:*

*Grenzkonzentrations-Test/Bodeninsekten*
Testinsekt: *Agrotis segetum*-Larven (im Boden)
Lösungsmittel: 3 Gew.-Teile Aceton
Emulgator: 1 Gew.-Teil Alkylarylpolyglykolether

Zur Herstellung einer zweckmässigen Wirkstoffzubereitung vermischt man 1 Gew.-Teil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und lässt diese bei Raumtemperatur stehen.

Nach 24 h werden die Testtiere in den behandelten Boden gegeben und nach weiteren 2 bis 7 d wird der Wirkungsgrad des Wirkstoffs durch Auszählen der toten und lebenden Testinsekten in Prozent bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 4, 5, 6, 9.

*Beispiel F:*

$LD_{100}$-Test für Dipteren
Testtiere: *Aedes aegypti*
Zahl der Testtiere: 20
Lösungsmittel: Aceton

2 Gew.-Teile Wirkstoff werden in 1000 Vol.-Teilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro Kubikmeter Filterpapier verschieden hoch. Anschliessend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen *knock down*-Effekt notwendig ist.

Bei diesem Test zeigen z.B. die folgenden Verbindungen der Herstellungsbeispiele überlegene Wirkung gegenüber dem Stand der Technik: 2, 4, 5, 6.

*Beispiel G:*

$LD_{100}$-Test
Testtiere: *Sitophilus granarius*
Zahl der Testtiere: 20
Lösungsmittel: Aceton

2 Gew.-Teile Wirkstoff werden in 1000 Vol.-Teilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro Kubikmeter Filterpapier verschieden hoch. Anschliessend gibt man die angegebene Anzahl der Testtiere in die Petrischale und bedeckt sie mit einem Glasdeckel.

Der Zustand der Testtiere wird 3 d nach Ansetzen der Versuche kontrolliert. Bestimmt wird die Abtötung in Prozent. Dabei bedeutet 100%, dass alle Testtiere abgetötet wurden; 0% bedeutet, dass keine Testtiere abgetötet wurden.

Bei diesem Test zeigen z.B. die folgenden Verbindungen der Herstellungsbeispiele überlegene Wirkung gegenüber dem Stand der Technik: 12, 7, 6, 5, 4, 2, 3.

*Beispiel H:*

*Test mit* Boophilus microplus *resistent*
Lösungsmittel: 35 Gew.-Teile Ethylenglykolmonomethylether
35 Gew.-Teile Nonylphenolpolyglykolether

Zur Herstellung einer zweckmässigen Wirkstoffzubereitung vermischt man 3 Gew.-Teile Wirkstoff mit 7 Gew.-Teilen des oben angegebenen Lösungsmittelgemisches und verdünnt das so erhaltene Konzentrat mit Wasser auf die gewünschte Konzentration.

10 adulte *Boophilus microplus* res. werden in die zu testende Wirkstoffzubereitung 1 min getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 1, 3, 6, 7, 9.

*Beispiel J:*

*Test mit* Lucilia cuprina *res.-Larven*
Emulgator: 35 Gew.-Teile Ethylenglykolmonomethylether
35 Gew.-Teile Nonylphenolpolyglykolether

Zur Herstellung einer zweckmässigen Wirkstoffzubereitung vermischt man 3 Gew.-Teile Wirkstoff mit 7 Gew.-Teilen des oben angegebenen Lösungsmitellgemisches und verdünnt das so erhaltene Konzentrat mit Wasser auf die jeweils gewünschte Konzentration.

Etwa 20 *Lucilia cuprina* res.-Larven werden in ein Teströhrchen gebracht, welches ca. 1 $cm^3$ Pferdefleisch und 0,5 ml der Wirkstoffzubereitung enthält. Nach 24 h wird der Abtötungsgrad bestimmt.

Bei diesem Test zeigen z.B. die folgenden Verbindungen der Herstellungsbeispiele überlegene Wirkung gegenüber dem Stand der Technik: 3, 6, 7, 9.

**Patentansprüche**

1. 3-Alken-1-yl-2,2-dimethylcyclopropancarbonsäure-(4-fluor-3-phenoxybenzyl)ester der Formel (I):

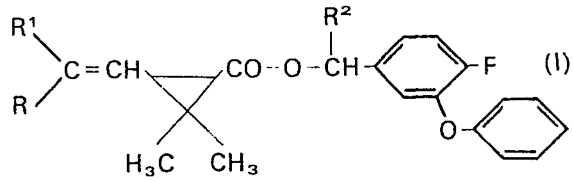
(I)

in welcher
R für Alkyl oder Alkenyl steht,
$R^1$ für Wasserstoff oder Alkyl steht mit der Massgabe, dass R und $R^1$ verschieden sind, und
$R^2$ für Wasserstoff, Cyano, Alkyl, Alkenyl oder Alkinyl, letztere jeweils mit bis zu 4 Kohlenstoffatomen, steht.

2. Verfahren zur Herstellung von 3-Alken-1-yl-2,2-dimethylcyclopropancarbonsäure-(4-fluor-3-phenoxybenzyl)estern der Formel (I):

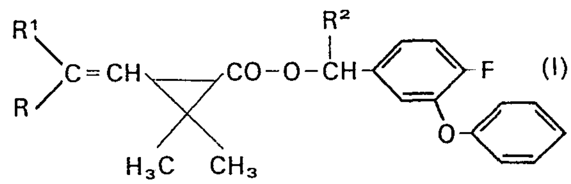
(I)

in welcher
R für Alkyl oder Alkenyl steht,
$R^1$ für Wasserstoff oder Alkyl steht mit der Massgabe, dass R und $R^1$ verschieden sind, und
$R^2$ für Wasserstoff, Cyano, Alkyl, Alkenyl oder Alkinyl, letztere jeweils mit bis zu 4 Kohlenstoffatome, steht,
dadurch gekennzeichnet, dass man 3-Alken-1-yl-2,2-dimethylcyclopropancarbonsäuren der Formel (II):

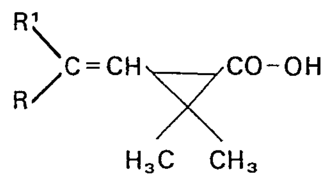
(II)

in welcher
R und $R^1$ die oben angegebene Bedeutung haben,
oder reaktionsfähige Derivate derselben, mit 4-Fluor-3-phenoxybenzylalkoholen der Formel (III):

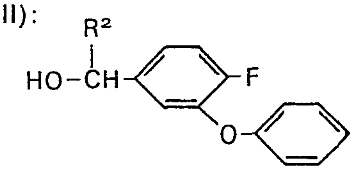
(III)

in welcher

$R^2$ die oben angegebene Bedeutung hat, oder mit reaktionsfähigen Derivaten derselben, gegebenenfalls in Gegenwart von Säureakzeptoren und/oder Katalysatoren und gegebenenfalls unter Verwendung von Verdünnungsmitteln umsetzt.

3. Verbindungen der Formel (I) gemäss Anspruch 1, in welcher R für $C_{1-25}$-Alkyl oder $C_{2-16}$-Alkenyl, $R^1$ für H oder $CH_3$ steht, mit der Massgabe, dass R und $R^1$ verschieden und $R^2$ für H oder CN steht.

4. Schädlingsbekämpfungsmittel, gekennzeichnet durch einen Gehalt an mindestens einem 3-Alken-1-yl-2,2-dimethylcyclopropancarbonsäure-(4-fluor-3-phenoxybenzyl)ester der Formel (I).

5. Verwendung von 3-Alken-1-yl-2,2-dimethylcyclopropancarbonsäure-(4-fluor-3-phenoxybenzyl)estern der Formel (I) zur Bekämpfung von Schädlingen.

6. Verfahren zur Bekämpfung von Schädlingen, dadurch gekennzeichnet, dass man 3-Alken-1-yl-2,2-dimethylcyclopropancarbonsäure-(4-fluor-3-phenoxybenzyl)ester der Formel (I) auf Schädlinge und/oder ihren Lebensraum einwirken lässt.

7. Verfahren zur Herstellung von Schädlingsbekämpfungsmitteln, dadurch gekennzeichnet, dass man 3-Alken-1-yl-2,2-dimethylcyclopropancarbonsäure-(4-fluor-3-phenoxybenzyl)ester der Formel (I) mit Streckmitteln und/oder oberflächenaktiven Mitteln vermischt.

## Claims

1. 4-Fluoro-3-phenoxybenzyl 3-alken-1-yl-2,2-dimethylcyclopropanecarboxylates of the formula (I):

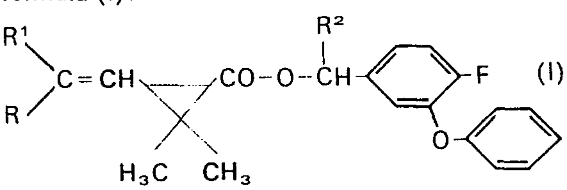
(I)

wherein

R represents alkyl or alkenyl,

$R^1$ represents hydrogen or alkyl, with the proviso that R and $R^1$ are different, and

$R^2$ represents hydrogen, cyano, alkyl, alkenyl or alkinyl, the latter each having up to 4 carbon atoms.

2. Process for the preparation of 4-fluoro-3-phenoxybenzyl 3-alken-1-yl-2,2-dimethylcyclopropanecarboxylates of the formula (I):

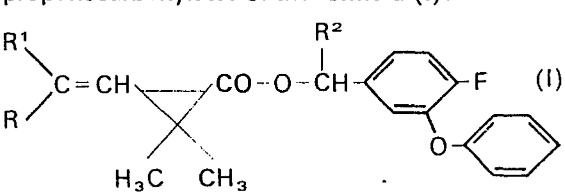
(I)

wherein

R represents alkyl or alkenyl,

$R^1$ represents hydrogen or alkyl, with the proviso that R and $R^1$ are different, and

$R^2$ represents hydrogen, cyano, alkyl, alkenyl or alkinyl, the latter each having up to 4 carbon atoms,

characterised in that 3-alken-1-yl-2,2-dimethylcyclopropanecarboxylic acids of the formula (II):

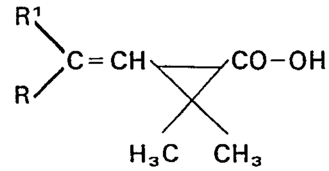
(II)

wherein

R and $R^1$ have the meaning given above, or reactive derivatives of these acids, are reacted with 4-fluoro-3-phenoxybenzyl alcohols of the formula (III):

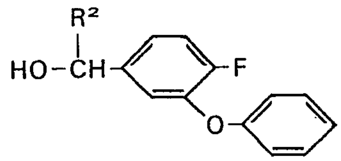
(III)

wherein

$R^2$ has the meaning given above, or with reactive derivatives of these alcohols, if appropriate in the presence of acid acceptors and/or catalysts, and if appropriate using diluents.

3. Compounds of the formula (I) according to Claim 1, wherein R represents $C_{1-25}$-alkyl or $C_{2-16}$-alkenyl, $R^1$ represents H or $CH_3$, with the proviso that R and $R^1$ are different, and $R^2$ represents H or CN.

4. Pest-combating agents, characterised in that they contain at least one 4-fluoro-3-phenoxybenzyl 3-alken-1-yl-2,2-dimethylcyclopropancarboxylate of the formula (I).

5. Use of 4-fluoro-3-phenoxybenzyl 3-alken-1-yl-2,2-dimethylcyclopropanecarbonxylates of the formula (I) for combating pests.

6. Process for combating pests, characterised in that 4-fluoro-3-phenoxybenzyl 3-alken-1-yl-2,2-dimethylcyclopropanecarbonxylates of the formula (I) are allowed to act on pests and/or their habitat.

7. Process for the preparation of pest-combating agents, characterised in that 4-fluoro-3-phenoxybenzyl 3-alken-1-yl-2,2-dimethylcyclopropanecarbonxylates of the formula (I) are mixed with extenders and/or surface-active agents.

## Revendications

1. Esters 4-fluoro-3-phénoxybenzyliques d'acides 3-alcène-1-yl-2,2-diméthylcyclopropanecarboxyliques de formule (I):

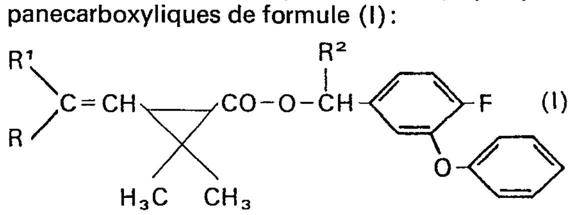
(I)

dans laquelle

R représente un groupe alkyle ou alcényle,

$R^1$ représente l'hydrogène ou un groupe alkyle, étant spécifié que R et $R^1$ sont différents, et

$R^2$ représente l'hydrogène, un groupe cyano, alkyle, alcényle ou alcynyle, ces derniers contenant chacun jusqu'à 4 atomes de carbone.

2. Procédé de préparation des esters 4-fluoro-3-phénoxybenzyliques d'acides 3-alcène-1-yl-2,2-diméthylcyclopropanecarboxyliques de formule (I):

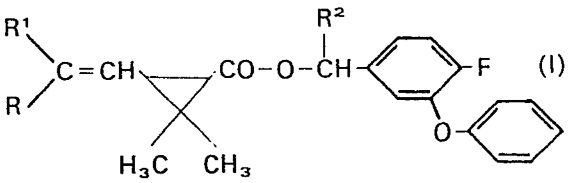
(I)

dans laquelle

R représente un groupe alkyle ou alcényle,

$R^1$ représente l'hydrogène ou un groupe alkyle, étant spécifié que R et $R^1$ sont différents,

$R^2$ représente l'hydrogène, un groupe cyano, alkyle, alcényle ou alcynyle, ces derniers contenant chacun jusqu'à 4 atomes de carbone, caractérisé en ce que l'on fait réagir des acides 3-alcène-1-yl-2,2-diméthylcyclopropanecarboxyliques de formule (II):

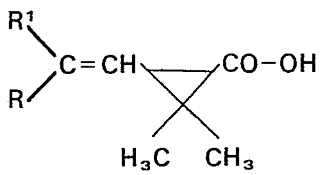
(II)

dans laquelle

R et $R^1$ ont les significations indiquées ci-dessus,

ou des dérivés réactifs de ces acides, avec des alcools 4-fluoro-3-phénoxybenzyliques de formule (III):

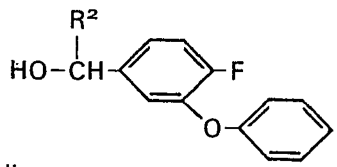
(III)

dans laquelle

$R^2$ a la signification indiquée ci-dessus,

ou avec les dérivés réactifs de ces alcools, éventuellement en présence d'accepteurs d'acides et/ou de catalyseurs et éventuellement en utilisant des diluants.

3. Composés de formule (I) selon la revendication 1, dans lesquels R représente un groupe alkyle en $C_1$-$C_{25}$ ou alcényle en $C_2$-$C_{16}$, $R^1$ représente H ou $CH_3$, étant spécifié que R et $R^1$ sont différents, et $R^2$ représente H ou CN.

4. Produit pesticide, caractérisé en ce qu'il contient au moins un ester 4-fluoro-3-phénoxybenzylique d'acide 3-alcène-1-yl-2,2-diméthylcyclopropanecarboxylique de formule (I).

5. Utilisation des esters 4-fluoro-3-phénoxybenzyliques d'acides 3-alcène-1-yl-2,2-diméthylcyclopropanecarboxyliques de formule (I) dans la lutte contre les parasites.

6. Procédé pour combattre des parasites, caractérisé en ce que l'on fait agir des esters 4-fluoro-3-phénoxybenzyliques d'acides 3-alcène-1-yl-2,2-diméthylcyclopropanecarboxyliques de formule (I) sur les parasites et/ou leur habitat.

7. Procédé de préparation de produits pesticides, caractérisé en ce que l'on mélange des esters 4-fluoro-3-phénoxybenzyliques d'acides 3-alcène-1-yl-2,2-diméthylcyclopropanecarboxyliques de formule (I) avec des diluants et/ou des agents tensio-actifs.